# EUROPEAN PATENT APPLICATION

(11) **EP 1 122 234 A2**
(43) Date of publication of application: **08.08.2001**
(21) Application number: 01102094.8
(22) Date of filing: 31.01.2001
(51) Int. Cl.: C07C 45/56, C07C 51/29, C07C 47/52, C07C 63/331

(54) **Production methods of alpha, alpha, alpha-trifluoromethylphenyl-substituted benzoic acid and intermediate therefor**

(30) Priority: 02.02.2000 JP 2000025328; 27.12.2000 JP 2000398504
(71) Applicant: SUMIKA FINE CHEMICALS Co., Ltd., Osaka-shi, Osaka 555-0021 (JP)
(72) Inventor: Shintaku, Tetsuya, c/o Sumika Fine Chem. Co., Ltd., Osaka-shi, Osaka 555-0021 (JP); Tanaka, Masahide, c/o Sumika Fine Chem. Co., Ltd., Osaka-shi, Osaka 555-0021 (JP); Shiratani, Hiroshi, c/oSumika Fine Chem. Co., Ltd., Osaka-shi, Osaka 555-0021 (JP); Itaya, Nobushige, c/o Sumika Fine Chem. Co., Ltd., Osaka-shi, Osaka 555-0021 (JP)
(74) Representative: von Kreisler, Alek, Dipl.-Chem.

(57) **Abstract**

The present invention relates to a production method of compound [V] useful as an intermediate for medicaments and agrochemicals. The method includes reacting compound [III] with hexamethylenetetramine under heating to give compound [IV], and oxidizing the obtained compound [IV] with a halous acid salt or a ruthenium compound. According to the present invention, moreover, an organometallic compound having a tolyl group and compound [I] are cross-coupled in the presence of a catalyst to give compound [II] useful as an intermediate for medicaments and agrochemicals. The compound [II] is halogenated to give compound [III]. 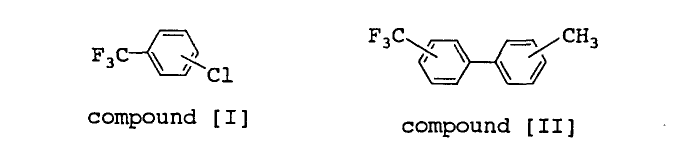 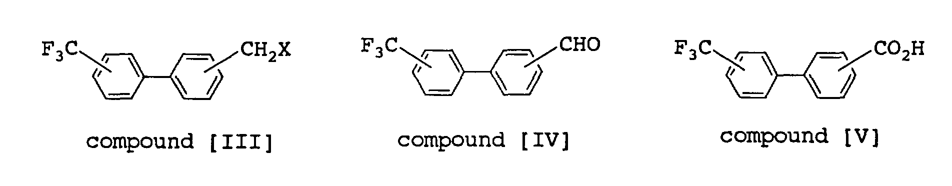 wherein X is halogen atom.

## Description

### TECHNICAL FIELD OF THE INVENTION

The present invention relates to a novel production method of a,a,a-trifluoromethylphenyl-substituted benzoic acid of the formula [V] below (hereinafter to be also referred to as compound [V]), which is represented by 2-[4-(trifluoromethyl)phenyl]benzoic acid useful as an intermediate for medicaments and agrochemicals. More particularly, the present invention relates to a novel production method of the compound [V] from α,α,α-trifluoromethylphenyl-substituted benzyl halide of the formula [III] below (hereinafter to be also referred to as compound [III]) as a starting material.

The present invention also relates to a production method of α,α,α-trifluoromethylphenyl-substituted benzaldehyde of the formula [IV] below (hereinafter to be also referred to as compound [IV]), which is a synthetic intermediate for compound [V].

Moreover, the present invention relates to a production method of a,a,a-trifluoromethylphenyl-substituted toluene of the formula [II] below (hereinafter to be also referred to as compound [II]), which is represented by 2-[4-(trifluoromethyl)phenyl]-toluene useful as an intermediate for medicaments and agrochemicals and which is useful and as a starting material of compound [III].

### BACKGROUND OF THE INVENTION

The production method of 2-[4-(trifluoromethyl)phenyl]-benzoic acid useful as an intermediate for medicaments (CP-467688 described in WO98/23593 and CP-319340 described in USP5919795, see the following formulas) 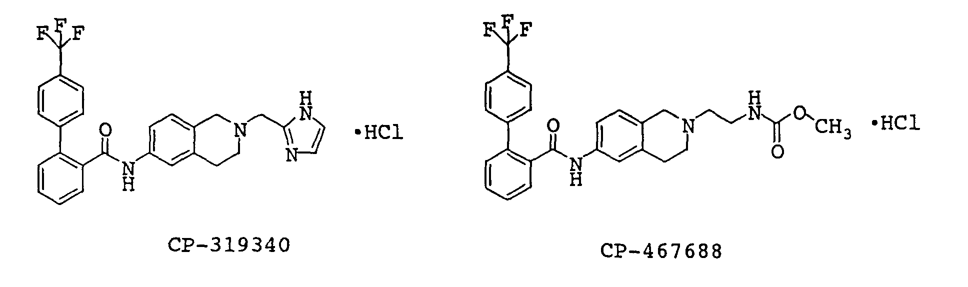 and agrochemicals comprises the following methods.
(1) Hydrolysis of 2-(4,4-dimethyl-2-oxazolin-2-yl)-4'-trifluoro-methylbiphenyl with hydrochloric acid (yield:46-86%, EP 59983 A1, USP 4578522 and Organic Preparation and Procedures Int., 27(3), 367-372(1995)).
(2) Oxidation of 4'-trifluoromethyl-2-biphenylcarbaldehyde (also referred to as 2-[4-(trifluoromethyl)phenyl]benzaldehyde) with potassium permanganate (yield:85%, EP 59983 A1 and USP 4578522).

2-(4,4-Dimethyl-2-oxazolin-2-yl)-4'-trifluoromethylbiphenyl used in the above-mentioned (1) can be obtained by adding 2-(2-methoxyphenyl)-4,4-dimethyloxazoline and magnesium to tetrahydrofuran, and adding p-bromobenzotrifluoride thereto to allow Grignard reaction (yield:33%, EP 59983 A1), or by reacting 2-(4,4-dimethyl-2-oxazolin-2-yl)phenyl zinc with 4-bromo (or chloro)benzotrifluoride using a nickel catalyst (nickel(I) catalyst prepared from nickel(II) compound by reacting diisobutylaluminum halide) to allow cross-coupling (Organic Preparation and Procedures Int., 27(3), 367-372(1995)). However, the former method shows a low yield of 33% and requires many steps, and the latter method requires complicated preparation of a catalyst. Thus, these production methods are not industrially useful.

4'-Trifluoromethyl-2-biphenylcarbaldehyde used in the above-mentioned (2) can be obtained by cross-coupling of 4-iodobenzotrifluoride and a Grignard reagent obtained from dimethylacetal derivative of 2-bromobenzaldehyde, in the presence of iodo(4-trifluoromethylphenyl)bis(triphenylphosphine)-palladium(II) as a catalyst, to give a biphenyl compound, which is then subjected to hydrolysis (deprotection) (yield:96.4%, EP 59983 A1 and USP 4578522). Although this method affords a high yield of 96%, an expensive palladium catalyst needs to be used. Thus, this method is not an industrially useful production method.

In view of the above, the present inventors desired to provide an economical, simple and easy, efficient and industrially useful production method of α,α,α-trifluoromethylphenyl-substituted benzoic acid of the formula [V] 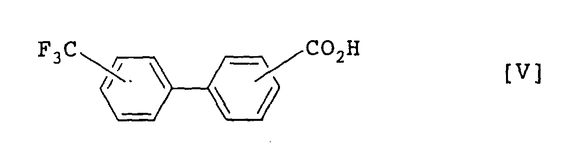 (compound [V]), through directly oxidizing α,α,α-trifluoromethylphenyl-substituted toluene of the formula [II] 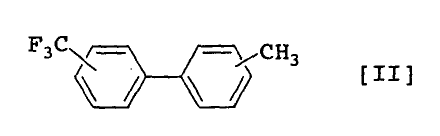 (compound [II]) and investigated the reaction conditions of this method. When compound [II] was reacted with potassium permanganate (KMnO₄) or ruthenium tetraoxide (RuO₄), both being representative oxidizing agents, the reactivity was lower than expected and only about 10% - 20% of the compound [II] was oxidized. Therefore, this method cannot produce compound [V] in a large amount and economically.

2-[4-(Trifluoromethyl)phenyl]toluene is useful as an intermediate for medicaments and agrochemicals, and as a starting material of compound [III]. Asymmetric biaryl compounds such as compound [II], which is represented by 2-[4-(trifluoromethyl)-phenyl]toluene, can be produced by cross-coupling reaction with an aryl halide and an aryl compound (e.g., arylmagnesium halide). When aryl halide contains a halogen which may be bromine or iodine, the cross-coupling reaction proceeds relatively easily. When halogen is chlorine, however, the cross-coupling reaction proceeds to give only an extremely low yield, while allowing easy progress of the homo-coupling reaction of aryl compound. Although aryl chloride shows defectively poor reactivity, it is economically advantageous. Thus, aryl chloride is preferably used as a starting material for industrial production. For this purpose, a high yield production method of compound [II] using aryl chloride as a starting material is desired.

In Tetrahedron Letters, 38(22), p3513-3516 (1997), aryl chloride is used as a starting material to produce compound [II] (see the following scheme). 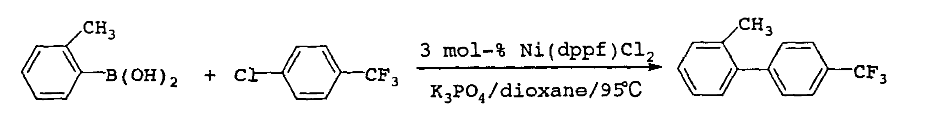 wherein dppf is 1,1'-bis(diphenylphosphino)ferrocene.

In this publication, arylboronic acid is used to allow reaction with aryl chloride. While arylboronic acid is commercially available, it is rather expensive. For industrial production, therefore, arylboronic acid is desirably produced by ourselves to avoid the high cost, wherein the use of arylboronic acid requires an increased number of steps to make the production complicated. In addition, the nickel catalyst, Ni(dppf)Cl₂, to be used alongside is expensive. Therefore, this method is not necessarily industrially preferable. In view of such situation, an economical, simple and easy production method of compound [II], which can be employed for industrial scale production has been desired.

It is therefore an object of the present invention to provide an economical, simple and easy, efficient and industrially useful production method of compound [V].

Another object of the present invention is to provide a simple and easy, industrially useful method for producing α,α,α-trifluoromethylphenyl-substituted benzaldehyde of the formula [IV] 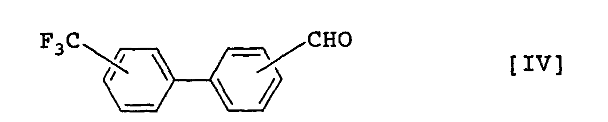 (compound [IV]), which is a synthetic intermediate for compound [V], economically in a high yield.

A yet another object of the present invention is to provide an economical, simple and easy production method of compound [II], which uses aryl chloride as a starting material, and which can be used for an industrial scale production.

### SUMMARY OF THE INVENTION

According to the present invention, compound [II] is not directly oxidized, but compound [II] is halogenated to give α,α,α-trifluoromethylphenyl-substituted benzyl halide of the formula [III] 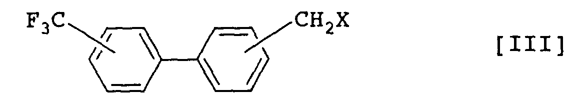 wherein X is halogen atom, (compound [III]). Compound [III] is then reacted with hexamethylenetetramine under heating. For a particularly high yield, formaldehyde or a polymer thereof is added and reacted. In this way, compound [IV], which is a synthetic intermediate for compound [V], can be produced in a high yield by an economical, simple and easy, and industrially useful method. In the context of the present invention, compound [IV] is oxidized with halous acid salt, whereby compound [V] can be produced by an economical, simple and easy, efficient and industrially useful method.

Moreover, for the production of compound [II], economical α,α,α-trifluoromethyl-substituted phenyl chloride of the formula [I] 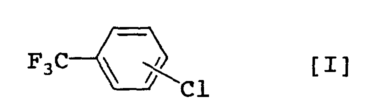 (hereinafter to be also referred to as compound [I]) is used, and an organometallic compound having a tolyl group, which can be prepared by a simple and easy method, is used and reacted therewith. As a result, the cross-coupling reaction preferentially proceeds, which enables economical production of compound [II] in a high yield by a simple and easy, industrially utilizable method, without use of an expensive catalyst.

Accordingly, the present invention provides a production method of compound [V], which includes the following steps (c) and (d).
step (c): reaction of compound [III] with hexamethylenetetramine with heating to give compound [IV].
step (d): oxidation of compound [IV] with a halous acid salt or ruthenium compound to give compound [V].

The preferable production method of compound [V] according to the present invention includes the following modes.
A) In step (c), formaldehyde or a polymer thereof is added.
B) In step (c), a formaldehyde polymer is added.
C) In the above-mentioned A) and B), formaldehyde or a polymer thereof is added in an amount of 0.1 g - 0.57 g per 1 g of compound [III].
D) The reaction of step (c) is carried out at pH 3.0 - 6.5.
E) In the reaction of step (c), acetic acid is added in an amount of 1 ml - 5 ml per 1 g of compound [III].
F) The reaction of step (d) using a chlorite as an oxidizing agent is carried out in a mixed solvent of t-butanol and water.
G) The halous acid salt in step (d) is a chlorite.
H) In the above-mentioned G), the chlorite is sodium chlorite.
I) The reaction of step (d) using a chlorite as an oxidizing agent is carried out in the presence of sulfamic acid.
J) The reaction of step (d) using a ruthenium compound as an oxidizing agent is carried out in the presence of at least one member selected from sodium hypochlorite, sodium periodate and sodium bromate.
K) The reaction of step (d) using a ruthenium compound as an oxidizing agent is carried out in the presence of a phase transfer catalyst in a two-phase system of water and a solvent immiscible with water.
L) The compound [III] is 2-[4-(trifluoromethyl)phenyl]benzyl bromide.

The compound [III] in the above-mentioned step (c) can be produced by halogenating compound [II] (step (b)).

The present invention also relates to a production method of compound [II], which comprises cross-coupling compound [I] and an organometallic compound having a tolyl group in the presence of a catalyst (step (a)). When an organometallic compound having a 2-methylphenyl group is used as the organometallic compound having a tolyl group, 2-methylphenyl-substituted α,α,α-trifluorotoluene of the formula [ii] 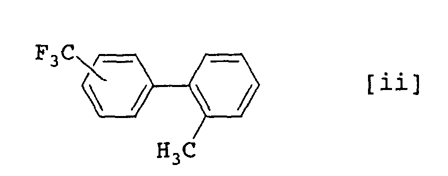 (hereinafter to be also referred to as compound [ii]) can be obtained. The preferable modes of the production method are as follows.
A) The compound [I] is 4-(trifluoromethyl)phenyl chloride.
B) The organometallic compound having a tolyl group is 2-methylphenylmagnesium halide.
C) The catalyst is a nickel catalyst.
D) The catalyst is bis(triphenylphosphine)nickel(II) dichloride.
E) The catalyst is a nickel catalyst, and a cocatalyst is also used.
F) In the above-mentioned E), the cocatalyst is [1] a zinc salt, or [2] a combination of a zinc salt and at least one member selected from a polar aprotic solvent and tertiary amine.
G) In the above-mentioned E) and F), the cocatalyst is a combination of a zinc salt and a polar aprotic solvent.
H) In the above-mentioned E) and F), the cocatalyst is a combination of a zinc salt and tertiary amine.
I) In the above-mentioned F)-H), the zinc salt is at least one member selected from zinc chloride and zinc bromide.
J) In the above-mentioned F) and G), the polar aprotic solvent is at least one member selected from N,N-dimethylformamide, dimethyl sulfoxide, N,N-dimethylacetamide and N-methyl-2-pyrrolidone.
K) In the above-mentioned F) and H), the tertiary amine is at least one member selected from N,N,N',N'-tetramethyl-ethylenediamine, N,N,N',N',N''-pentamethyldiethylene-triamine, dimethylaniline and pyridine.
L) In the above-mentioned E), the catalyst is bis(triphenylphosphine) nickel(II) dichloride and the cocatalyst is a combination of zinc chloride and N,N-dimethylacetamide.
M) In the above-mentioned E), the catalyst is bis(triphenylphosphine) nickel(II) dichloride and the cocatalyst is a combination of zinc chloride and N,N,N',N'-tetramethylethylenediamine.

### DETAILED DESCRIPTION OF THE INVENTION

The present invention is explained in detail in the following.

The halogen atom at X in the formula [III] is exemplified by chlorine atom, bromine atom and iodine atom. In view of the reactivity and easiness of preparation, bromine atom is particularly preferable.

With regard to compound [I] of the present invention, the position of chlorine atom and trifluoromethyl group, which are substituents, is not particularly limited. For example, 4-(trifluoromethyl)phenyl chloride, 3-(trifluoromethyl)phenyl chloride, 2-(trifluoromethyl)phenyl chloride and the like are exemplified. Preferably, 4-(trifluoromethyl)phenyl chloride is used.

The compound [II] in the present invention includes position isomers at any position. Examples of preferable position isomer include 2-[4-(trifluoromethyl)phenyl]toluene, 3-[4-(trifluoromethyl)phenyl]toluene, 4-[4-(trifluoromethyl)phenyl]-toluene, 2-[3-(trifluoromethyl)phenyl]toluene, 2-[2-(trifluoromethyl)phenyl]toluene and the like. Of these, 2-[4-(trifluoromethyl)phenyl]toluene and 2-[3-(trifluoromethyl)phenyl]-toluene are preferable. Particularly, 2-[4-(trifluoromethyl)-phenyl]toluene is preferable.

The compound [III] in the present invention includes position isomers at any position. Examples of preferable position isomer include 2-[4-(trifluoromethyl)phenyl]benzyl bromide, 3-[4-(trifluoromethyl)phenyl]benzyl bromide, 4-[4-(trifluoromethyl)-phenyl]benzyl bromide and the like, particularly preferably 2-[4-(trifluoromethyl)phenyl]benzyl bromide.

The compound [IV] in the present invention includes position isomers at any position. Examples of preferable position isomer include 2-[4-(trifluoromethyl)phenyl]benzaldehyde, 3-[4-(trifluoromethyl)phenyl]benzaldehyde, 4-[4-(trifluoromethyl)-phenyl]benzaldehyde and the like, particularly preferably 2-[4-(trifluoromethyl)phenyl]benzaldehyde.

The compound [V] in the present invention includes position isomers at any position. Examples of preferable position isomer include 2-[4-(trifluoromethyl)phenyl]benzoic acid, 3-[4-(trifluoromethyl)phenyl]benzoic acid, 4-[4-(trifluoromethyl)-phenyl]benzoic acid and the like, particularly preferably 2-[4-(trifluoromethyl)phenyl]benzoic acid.

### step (a) : Production of compound [II] from compound [I]

In step (a), compound [II] (particularly compound [ii]) can be produced by cross-coupling of compound [I] and an organometallic compound having a tolyl group in the presence of a catalyst. For example, compound [I] is added to a reaction solvent and a catalyst is added, which is followed by addition, preferably dropwise addition, of the above-mentioned organometallic compound, to allow the progress of the cross-coupling reaction. The compound [I] used in step (a) is commercially available and rather economical as compared to the corresponding bromine compound.

The organometallic compound having a tolyl group is preferably an organometallic compound having a 2-methylphenyl group. As used herein, by the organometallic compound having a 2-methylphenyl group is meant an organometallic compound having a phenyl group as a base, wherein a group having a metal atom is substituted at the 1-position, and a methyl group is substituted at the 2-position. The metal of the above-mentioned organometallic compound used in step (a) does not include an element showing less metallic property (semi-metal), such as boron and silicon. The metal is exemplified by lithium, magnesium, zinc and the like, preferably magnesium. Examples of the organometallic compound having a 2-methylphenyl group include 2-methylphenyllithium, 2-methylphenylmagnesium halide (e.g., 2-methylphenylmagnesium chloride, 2-methylphenylmagnesium bromide, 2-methylphenylmagnesium iodide), 2-methylphenylzinc chloride and the like, with preference given to 2-methylphenylmagnesium halide, because they can be easily prepared. This organometallic compound is used in an amount of preferably 0.9 - 2.0 equivalents, more preferably 1.0 - 1.5 equivalents, relative to compound [I].

The catalyst used in step (a) may be any as long as it is generally used for cross-coupling reaction, such as nickel catalyst (e.g., bis(triphenylphosphine)nickel(II) dichloride, nickel(II) dichloride, etc.), manganese catalyst (e.g., manganese chloride, a catalyst obtained from manganese dioxide and chlorotrimethylsilane, etc.) and the like, preferably a nickel catalyst. Of the nickel catalysts, bis(triphenylphosphine)nickel-(II) dichloride is particularly preferable, because it affords a high yield and is obtained relatively easily. The catalyst is used in an amount of preferably 0.05 mol% (equivalent %) - 20 mol% (equivalent %), more preferably 0.1 mol% (equivalent %) - 10 mol% (equivalent %), relative to compound [I].

When the catalyst is a nickel catalyst in step (a), a cocatalyst is preferably used along with the catalyst to make the reaction proceed smoothly and to suppress a side reaction. Examples of the cocatalyst include [1] zinc salt and [2] a combination of a zinc salt and at least one member selected from polar aprotic solvents and tertiary amine. In other words, the cocatalyst may be (1) a zinc salt or (2) a combination of a zinc salt and a polar aprotic solvent, (3) a combination of zinc salt and tertiary amine, or (4) a combination of zinc salt, a polar aprotic solvent and tertiary amine. The zinc salt may be, for example, zinc chloride and zinc bromide, preferably zinc chloride. Examples of the polar aprotic solvent include N,N-dimethyl-formamide, dimethyl sulfoxide, N,N-dimethylacetamide and N-methyl-2-pyrrolidone, preferably N,N-dimethylacetamide. Examples of the tertiary amine include N,N,N',N'-tetramethylethylenediamine, N,N,N',N',N''-pentamethyldiethylenetriamine, dimethylaniline, pyridine and the like, preferably N,N,N',N'-tetramethylethylene-diamine. The zinc salt, polar aprotic solvent and tertiary amine may be respectively used alone or in combination. When a catalyst and a cocatalyst are used, the amount of the catalyst is the same as that when used alone, which is preferably 0.05 mol% (equivalent %) - 20 mol% (equivalent %), more preferably 0.1 mol% (equivalent %) - 10 mol% (equivalent %), relative to compound [I], an the amount of the cocatalyst is preferably 5 mol% (equivalent %) - 50 mol% (equivalent %) relative to compound [I]. The amount of the above-mentioned cocatalyst is the amount of each cocatalyst when two or more kinds of the cocatalysts are used.

When the reaction is carried out in the presence of a catalyst and a cocatalyst, a combination of, for example, the catalyst being bis(triphenylphosphine)nickel(II) dichloride and the cocatalyst being a combination of zinc chloride and N,N-dimethylacetamide; and the catalyst being bis(triphenylphosphine)nickel(II) dichloride and the cocatalyst being a combination of zinc chloride and N,N,N',N'-tetramethylethylenediamine are particularly preferable.

The reaction solvent in step (a) is not particularly limited as long as it does not interfere with the reaction. It is preferably an ether solvent, or a mixed solvent of an ether solvent and a different solvent inert to the reaction of step (a). The reaction solvent is preferably substantially an ether solvent, but the solvent other than the ether solvent may be used in an amount that does not impair the object of the present invention. The amount of the reaction solvent to be used varies depending on the kind and amount of the sample materials used for the reaction. The lower limit is generally not less than 1.5-fold, preferably not less than 2-fold, more preferably not less than 3-fold, particularly preferably not less than 4-fold, and the upper limit is generally not more than 30-fold, preferably not more than 15-fold, preferably 3-fold to 30-fold, particularly preferably 4-fold to 15-fold, of the weight of compound [I].

The above-mentioned ether solvent may be tetrahydrofuran, diisopropyl ether, t-butyl methyl ether and the like, preferably tetrahydrofuran (THF). These can be used alone or in combination.

The solvent other than the above-mentioned ether solvent, which is inert to the reaction of step (a), may be an organic solvent that does not react with the organometallic compound (e.g., Grignard reagent and the like) used in step (a), such as aromatic hydrocarbon (e.g., toluene, xylene, etc.).

The reaction temperature and reaction time in step (a) varies depending on the kind and amount of the solvent, catalyst, starting material, cocatalyst and the like. The reaction temperature is generally 0°C - 70°C, preferably 10°C - 60°C, and the reaction time is 30 minutes - 20 hours, preferably 3 hours-15 hours. While the dropwise addition of the organometallic compound varies depending on the amount thereof, it is generally conducted over 30 minutes - 10 hours.

The reaction of step (a) is preferably carried out in an inert gas atmosphere, such as nitrogen gas and argon gas. The pressure of the above-mentioned inert gas is not particularly limited and may be atmospheric pressure.

The compound [II] can be isolated by a conventional method. For example, the reaction mixture is partitioned using hydrochloric acid and the like, the obtained organic layer is washed with brine and the like, concentrated and distilled to isolate compound [II]. By treating with active charcoal, silica gel, alumina and the like, the purity of compound [II] can be increased.

The organometallic compound having a tolyl group used in step (a), such as an organometallic compound having a 2-methylphenyl group, can be generally produced simply and easily according to the production method of an organometallic compound. For example, 2-methylphenylmagnesium halide can be generally produced by the similar method as the preparation of Grignard reagent, by reacting o-halotoluene and metal magnesium.

The obtained compound [II] can be introduced into compound [V] which is useful as a synthetic intermediate for medicaments and agrochemicals via steps (b), (c) and (d) of the present invention. The compound [II] can be also introduced into a useful medicament, such as CP-319340 and CP-467688, according to the method of USP 5919795, WO 9640640, EP 944602, WO 9823593 and the like, after converting methyl group to carboxyl group by a conventional method.

### step (b) : production of compound [III] from compound [II]

In step (b), compound [II] is halogenated to give compound [III]. The halogenation is most preferably bromination, in view of the reactivity of halogenating agent and easiness of operation. The halogenation is conducted using a halogenating agent.

The halogenating agent used in step (b) is not particularly limited as long as the reaction proceeds, and a typical halogenating agent, such as sulfuryl chloride, N-bromosuccinimide (NBS), bromine, 1,3-dibromo-5,5-dimethylhydantoin and the like, is used. In view of the reactivity and easiness of the operation, it is preferably a brominating agent, and in view of the economic aspect and operability, bromine is particularly preferable.

The amount of the halogenating agent varies depending on the kind and the like of the halogenating agent. When bromine is used as the halogenating agent, the amount thereof is preferably 1 mol - 1.7 mol, more preferably 1.1 mol - 1.5 mol, per 1 mol of compound [II].

The reaction solvent in step (b) is not particularly limited as long as it does not interfere with the halogenation in step (b). Preferred is monochlorobenzene. The amount of the reaction solvent to be used is preferably 2 g - 15 g, more preferably 3 g-10 g, per 1 g of compound [II].

In step (b), the reaction temperature is preferably 20°C-90°C, more preferably 40°C - 70°C, and the reaction time is preferably 3 hours - 15 hours, more preferably 5 hours - 10 hours.

The order of addition of compound [II] and halogenating agent is not particularly limited. For example, compound [II] is added to a reaction solvent, and then a halogenating agent is added, preferably added dropwise or added by portions, to give compound [III].

It is also preferable to add a catalyst amount of a radical initiator (e.g., azobisisobutyronitrile (AIBN) and the like) to the reaction system.

When compound [II] is halogenated, compound [III'] of the following formula [III'] 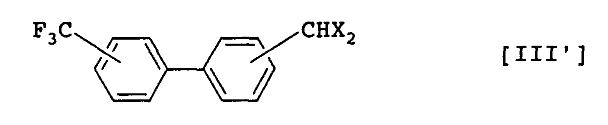 wherein X is halogen atom, may be produced besides compound [III].

The compound [III] can be isolated by a conventional method. For example, after the completion of the reaction, the reaction mixture is concentrated under reduced pressure and the residue is subjected to crystallization to isolate compound [III]. The compound [III] can be purified by a conventional method, such as recrystallization, column chromatography and the like. When compound [III'] is simultaneously produced besides compound [III], these may be used for the production of compound [IV], without separation.

### step (c): production of compound [IV] from compound [III]

In step (c), compound [III] is reacted with hexamethylenetetramine with heating to give compound [IV] (Sommelet reaction).

Hexamethylenetetramine is used in step (c) in an amount of 2 mol - 6 mol per 1 mol of compound [III]. From the economical aspect, it is preferably 3 mol - 4 mol.

In general, the Sommelet reaction is carried out by reacting hexamethylenetetramine with alkyl halide (inclusive of aryl-substituted alkyl halide). For an improved yield, formaldehyde or a polymer thereof is preferably added to the reaction system. Of these, paraformaldehyde, which is a polymer of formaldehyde, is particularly preferably added because of the superior handling property it affords. Paraformaldehyde can be easily decomposed into formaldehyde under the heating conditions in the Sommelet reaction.

Formaldehyde or a polymer thereof is added in an amount of generally 0.1 g- 0.57 g per 1 g of compound [III], and preferably 0.25 g- 0.38 g from the economical aspect.

When aldehyde is obtained in the Sommelet reaction, the reaction is reported to be conducted at a pH of 3.0 - 6.5 (Organic Reactions, 8, 197-217(1954)). Therefore, the above-mentioned pH is preferably employed by adjusting by the use of an acid, such as mineral acid, acetic acid and the like. The use of acetic acid is preferable, because the reaction can be carried out without adjusting the pH during the reaction to the above-mentioned range. In general, acetic acid is preferably added in an amount of 1 ml-5 ml, industrially 1 ml - 3 ml, per 1 g of compound [III].

The reaction solvent in step (c) is not particularly limited as long as it does not interfere with the reaction. In general, hydrocarbons (e.g., toluene and the like), halogenated hydrocarbons (e.g., monochlorobenzene and the like), lower alcohol, esters (e.g., ethyl acetate and the like) and the like are used, particularly preferably monochlorobenzene. In addition, an acid such as acetic acid and the like mentioned above may act as a reaction solvent. These reaction solvents may be used alone or in combination. While the amount of the reaction solvent is not particularly limited, when a single reaction solvent is used, it is generally preferably 1 ml - 7 ml, industrially 1.5 ml - 6 ml, per 1 g of compound [III]. When two or more kinds thereof are used in combination, each is used in an amount of preferably 1 ml - 7 ml, industrially 1.5 ml - 6 ml, per 1 g of compound [III].

In step (c), the reaction time and the reaction temperature are closely related and a general definition thereof is difficult to give. When the reaction time is 1 hour - 3 hours, for example, the reaction is carried out at generally 50°C - 95°C.

The order of addition of compound [III] and hexamethylenetetramine is not particularly limited. When the reaction is carried out in a system where paraformaldehyde is added, for example, hexamethylenetetramine is dissolved in a reaction solvent, then compound [III] is added, preferably added or added dropwise after dissolution in a solvent, and paraformaldehyde is added, which is followed by stirring with heating to give compound [IV]. The solvent to be used for dissolving compound [III] is preferably the above-mentioned reaction solvent, particularly preferably monochlorobenzene.

The compound [IV] can be isolated by routine steps, such as extraction, distillation, column chromatography and the like. The compound [IV] is purified by a conventional method.

### step (d) : production of compound [V] from compound [IV]

In step (d), compound [IV] is oxidized to give compound [V]. In this step, halous acid salt or ruthenium compound is used as an oxidizing agent. In comparison with the use of potassium permanganate, manganese dioxide produced by the reaction does not need to be removed and the post-reaction treatment is advantageously facilitated.

The step (d) is explained in the following according to the case where a halous acid salt is used as an oxidizing agent and the case where a ruthenium compound is used as an oxidizing agent. When a halous acid salt is used

The halous acid salt to be used in step (d) may be chlorite or bromite, with preference given to chlorite from the economical aspect. The halous acid salt may be a salt of halous acid with an alkali metal such as sodium, potassium and the like, with preference given to sodium salt considering the availability. Examples of the halous acid salt include sodium chlorite, potassium chlorite, sodium bromite, potassium bromite and the like. In consideration of the economic aspect and availability, sodium chlorite is particularly preferable. The amount of halous acid salt to be used is generally 1 mol - 5 mol, and from the economic aspect, preferably 2 mol - 3 mol, per 1 mol of compound [IV].

Sodium chlorite can be generally used in the form of a 80% solid powder or a 25% aqueous solution. For industrial use, it is preferably in the form of a 25% aqueous solution in view of handling property, safety and the like.

When a halous acid salt is used, step (d) is preferably carried out in the presence of sulfamic acid (Acta Chemica Scandinavica, 27, 888-890 (1973)). The amount of the sulfamic acid to be used is generally 1 mol - 5 mol, and from the economic aspect, preferably 2 mol - 3 mol, per 1 mol of compound [IV].

When a halous acid salt is used in step (d), the reaction solvent can be, for example, water, lower alcohol, acetonitrile, hydrocarbons (e.g., toluene and the like), or a mixed solvent thereof. Examples of the lower alcohol include methanol, ethanol, t-butanol, 1-butanol and the like, with preference given to t-butanol, because it is hardly oxidized. It is also preferable to use water for dissolving sulfamic acid in the reaction system. In the present invention, the use of a mixed solvent of t-butanol and water is preferable. The amount of the reaction solvent used is not particularly limited. When a single reaction solvent is used, it is generally 1 ml - 5 ml, and from the economic aspect, preferably 2 ml - 4 ml, per 1 g of compound [IV]. When the solvents are used in combination, the amount of each solvent is generally 1 ml - 5 ml, and from the industrial aspect, preferably 2 ml - 4 ml, per 1 g of compound [IV].

When a halous acid salt is used in step (d), the reaction temperature is generally 0°C - 30°C, preferably 0°C - 10°C, and the reaction time is generally 0.5 hour - 3 hours, preferably 1 hour-2 hours.

When a halous acid salt is used in step (d), the reaction system can be maintained at a constant pH by the use of dihydrogenphosphate, such as potassium dihydrogenphosphate, sodium dihydrogenphosphate and the like. While the amount varies depending on the compound, the amount of dihydrogenphosphate is preferably not less than 1 mol, and from the economic aspect, more preferably 1 mol - 3 mol, per 1 mol of compound [IV].

The compound [V] can be isolated by, after the completion of the reaction, treating the residual halous acid salt with a reducing agent (e.g., sodium bisulfite, sodium thiosulfate and the like), followed by partitioning, concentration under reduced pressure and crystallization. The compound [V] is purified by a conventional method.

### When a ruthenium compound is used

Examples of the ruthenium compound to be used in step (d) include anhydrous ruthenium(III) chloride (RuCl₃) and hydrate thereof, ruthenium(III) acetylacetonate (Ru(acac)₃), anhydrous ruthenium(III) bromide (RuBr₃) and hydrate thereof, ruthenium(III) iodide (RuI₃), anhydrous ruthenium(IV) oxide (RuO₂) and hydrate thereof, ruthenium(VIII) oxide (RuO₄), potassium ruthenate (K₂RuO₄) and the like, with preference given to anhydrous ruthenium(III) chloride and hydrate thereof.

In step (d), when a ruthenium compound is used as an oxidizing agent to oxidize compound [IV], a large amount of the ruthenium compound is required. The present inventors have studied the reduction of the amount of the ruthenium compound in step (d), and found that the coexistence of an oxidizing agent, such as sodium hypochlorite, sodium periodate, sodium bromate and the like, enables oxidation of a reductant (ruthenium compound) produced by the oxidation reaction in the reaction system, and that the oxidized compound can be used again as an active oxidizing agent (ruthenium compound) for the oxidation of compound [IV]. In addition, compound [V] can be obtained in a high yield. The amount of the ruthenium compound to be used in step (d), where an oxidizing agent, such as sodium hypochlorite and the like, is co-used, is generally 0.1 mol - 100 mol, preferably 0.9 mol - 1.2 mol, per 100 mol of compound [IV].

The oxidizing agent to be used concurrently may be any as long as it can oxidize a reduced ruthenium compound, preferably sodium hypochlorite, sodium periodate and sodium bromate, with particular preference given to sodium hypochlorite. These oxidizing agents may be used alone or in combination. The amount of the oxidizing agent is generally not less than 1.0 mol per 1 mol of compound [IV], and from an economic aspect, 1.1 mol - 5 mol is preferable.

The step (d) using a ruthenium compound is performed in a two-phase system of water and a solvent immiscible with water, and the solvent immiscible with water is not particularly limited as long as it dissolves compound [IV] and it does not interfere with the oxidation reaction in step (d). Examples thereof include monochlorobenzene, dichlorobenzene, carbon tetrachloride, chloroform, dichloromethane, dichloroethane and the like, with preference given to monochlorobenzene. The amount of the solvent immiscible with water is not particularly limited as long as it dissolves compound [IV]. It is an amount that makes the concentration of compound [IV] generally about 1 wt% - 50 wt%, preferably 20 wt% - 50 wt%. The amount of water is not particularly limited. It is an amount that makes the concentration of the oxidizing agent to be used concurrently, such as sodium hypochlorite and the like, generally about 5 wt% - 20 wt%, preferably 5 wt% - 15 wt%. For example, sodium hypochlorite is generally commercially available in the form of an aqueous solution, and water in the aqueous solution is considered to be the water of the solvent in step (d).

The step (d) using a ruthenium compound is conducted in the presence of a phase transfer catalyst. Examples of the phase transfer catalyst include tetrabutylammonium bromide, tetrabutylammonium chloride, tetrabutylammonium iodide, tetrabutylammonium hydrogensulfate, benzyltrimethylammonium bromide, benzyltrimethylammonium chloride, cetyltrimethylammonium bromide, cetyltrimethylammonium chloride and the like, with preference given to tetrabutylammonium bromide. The amount of the phase transfer catalyst to be used is generally about 2 mol - 10 mol, preferably 4 mol - 10 mol, per 100 mol of compound [IV].

In step (d) using a ruthenium compound, the reaction temperature is generally 0°C - 100°C, preferably 10°C - 50°C, and the reaction time is generally 30 minutes - 20 hours, preferably 1 hour - 8 hours.

The compound [V] can be isolated and purified by a conventional method. For example, the reaction mixture is acidified and extracted with an organic solvent to isolate compound [V]. After the isolation, it is applied to column chromatography and the like for purification.

The compound [V] can be led to CP-467688 by the method described in WO 98/23593, and to CP-319340 by the method described in USP 5919795.

The present invention is explained in more detail in the following by referring to examples which do not limit the present invention.

### Example 1 (production of compound [II] from compound [I] using zinc salt as a cocatalyst)

In a 500 ml four-neck flask, THF (177 g), p-chloro-α,α,α-trifluorotoluene (54.2 g, 0.30 mol) and Ni(PPh₃)₂Cl₂ (wherein Ph is phenyl group; 0.9 g, 1.15 mmol) were charged. Separately, 2-methylphenylmagnesium chloride (55.8 g) was obtained as a THF solution (121 g) by a conventional method, and without isolation, added dropwise thereto under a nitrogen atmosphere at 20°C - 30°C over 2 hr. After the dropwise addition, the mixture was stirred for 2 more hours. 1N Hydrochloric acid (200 ml) was added to the reaction mixture to partition the mixture. The obtained organic layer was washed with saturated brine (100 ml). The organic layer was concentrated under reduced pressure and distilled to give 2-[4-(trifluoromethyl)phenyl]toluene (56.7 g, 0.24 mol, yield 80%) as a colorless oil.
¹H-NMR(400MHz,CDCl₃)δ:
7.66(brd,J=8.3Hz,2H), 7.42(brd,J=8.3Hz,2H), 7.28-7.18(m,4H),
2.25(d,J=3.4Hz,3H).

### Example 2 (production of compound [II] from compound [I] using a combination of zinc salt and polar aprotic solvent as a cocatalyst)

In a 300 ml four-neck flask, THF (40 ml), p-chloro-α,α,α-trifluorotoluene (4.7 g, 0.026 mol) and Ni(PPh₃)₂Cl₂ (wherein Ph is phenyl group; 0.68 g, 4 mol% relative to p-chloro-α,α,α-trifluorotoluene), ZnCl₂ (0.57 g, 16 mol% relative to p-chloro-a,a,a-trifluorotoluene) and N,N-dimethylacetamide (0.36 g, 16 mol% relative to p-chloro-α,α,α-trifluorotoluene) were charged. Separately, 2-methylphenylmagnesium chloride (4.8 g) was obtained as a THF solution (10.5 g) by a conventional method, and without isolation, added dropwise thereto under a nitrogen atmosphere at 20°C - 30°C over 2 hr. After the dropwise addition, the mixture was stirred for 2 more hours. Water (8.2 g) and 35% hydrochloric acid (4.1 g) were added to the reaction mixture and the mixture was extracted with toluene (15 ml). The obtained organic layer was concentrated under reduced pressure and distilled to give 2-[4-(trifluoromethyl)phenyl]toluene (5.16 g, 0.022 mol, yield 84%) as a colorless oil. The ¹H-NMR of the obtained colorless oil was the same as in Example 1.

### Example 3 (production of compound [II] from compound [I] using a combination of zinc salt and tertiary amine as a cocatalyst)

In a four-neck flask, THF (5.02 g), p-chloro-α,α,α-trifluorotoluene (5.03 g, 27.7 mmol), Ni(PPh₃)₂Cl₂ (wherein Ph is phenyl group; 0.91 g, 5 mol% relative to p-chloro-α,α,α-trifluorotoluene), N,N,N',N'-tetramethylethylenediamine (0.32 g, 2.8 mmol, 10 mol% relative to p-chloro-α,α,α-trifluorotoluene) and ZnCl₂ (0.38 g, 2.8 mmol, 10 mol% relative to p-chloro-a,a,a-trifluorotoluene) were successively charged. Separately, 2-methylphenylmagnesium chloride (5.0 g) was obtained as a THF solution (10.9 g) by a conventional method, and without isolation, added dropwise thereto under a nitrogen atmosphere at 20°C - 30°C over 2 hr. After the dropwise addition, the mixture was stirred overnight. The reaction mixture was added dropwise to an aqueous hydrochloric acid solution (containing water (15 g) and 35% hydrochloric acid (1.0 g)) and the mixture was partitioned. The obtained organic layer was analyzed. As a result, the content of 2-[4-(trifluoromethyl)phenyl]toluene) in the organic layer was 4.57 g (yield:70%).

### Example 4 (production of compound [IV] from compound [III])

Hexamethylenetetramine (1.90 g, 13.6 mmol) was added and dissolved in acetic acid (1.5 ml). Then, a monochlorobenzene solution (7 g) containing 2-[4-(trifluoromethyl)phenyl]benzyl bromide (1 g, 3.17 mmol) was added dropwise. Paraformaldehyde (0.38 g) was added and the mixture was stirred at 87°C - 92°C for 1 hr. Deionized water (2 ml) and toluene (2 ml) were added for partitioning and the obtained organic layer was analyzed by the LC-IS method. As a result, the amount of 2-[4-(trifluoromethyl)phenyl]benzaldehyde was 0.73 g (yeild:91.9%).
¹H-NMR(400MHz, CDCl₃)δ:
9.93(s, 1H), 8.01(d,J=7.3Hz,lH), 7.72(d,J=8.3Hz,2H),
7.64(t, J=7.6Hz, 1H), 7.53-7.48(m,2H), 7.41(d, J=7.3Hz, 1H).

### Example 5 (production of compound [V] from compound [IV] using a halous acid salt

To 2-[4-(trifluoromethyl)phenyl]benzaldehyde (0.73 g, 2.92 mmol) obtained in Example 1 were added t-butanol (1.5 ml), deionized water (1.5 ml) and sulfamic acid (0.42 g, 4.33 mmol). Thereto was added dropwise a 25% aqueous sodium chlorite solution (1.58 g, 4.37 mmol) under ice-cooling. The mixture was stirred for 30 min and sulfamic acid (0.28 g, 2.88 mmol) was added. Then, a 25% aqueous sodium chlorite solution (1.06 g, 2.93 mmol) was added dropwise and the mixture was stirred for 30 min. Toluene (5 ml) and deionized water (5 ml) were added for partitioning and the obtained organic layer was concentrated to give 2-[4-(trifluoromethyl)phenyl]benzoic acid (0.73 g, yield:94.0%).
¹H-NMR(400MHz,CDCl₃)δ:
8.02(dd,J=7.8,1.0Hz,1H), 7.63(d,J=7.8Hz,2H),
7.53(ddd,J=7.8,7.8,1.5Hz,1H), 7.47(ddd,J=7.8,7.8,1.5Hz,1H),
7.41(d,J=7.8Hz,2H), 7.32(dd,J=7.8,1.0Hz,1H).
¹³C-NMR(100MHz,CDCl₃)δ:
172.4, 144.7, 142.2, 132.3, 131.0(2C), 128.7(2C), 127.8,
129.4(²J(C_{4'},F)=32.3), 124.1(¹J(CF₃,F)=227.0),
124.8(³J(C_{3'},F)=³J(C_{5'},F)=3.3).
FT-IR(KBr)ν:3015, 1684, 1320.
mp:169.1°C - 170.2°C

### Example 6 (production of compound [V] from compound [IV] using a ruthenium compound)

To a solution of 2-[4-(trifluoromethyl)phenyl]benzaldehyde (1.0 g, 4.0 mmol) in monochlorobenzene (2 ml) were added ruthenium(III) chloride trihydrate (12.8 g, 0.049 mmol) and tetran-butylammonium bromide (60 mg, 0.186 mmol). Further, a 5% aqueous sodium hypochlorite solution (27 g, 18.1 mmol) was added and the mixture was stirred at room temperature for 1 hr. The reaction mixture was acidified with 10% sulfuric acid and extracted with ethyl acetate to give a solution of 2-[4-(trifluoromethyl)phenyl]benzoic acid in ethyl acetate. The content of benzoic acid compound in this solution was 0.753 g (yield:70.8%) as determined by LC analysis (internal standard method).

### Example 7 (comparison of use or non-use of paraformaldehyde in the production of compound [IV] from compound [III])

Hexamethylenetetramine (1.35 g, 9.63 mmol) was added and dissolved in acetic acid (1.5 ml). Then, a monochlorobenzene solution (7 g) containing 2-[4-(trifluoromethyl)phenyl]benzyl bromide (1 g, 3.17 mmol) was added dropwise. Thereafter, paraformaldehyde (0.28 g) was added and the mixture was stirred at 58°C - 62°C for 1 hr. Deionized water (2 ml) and toluene(2 ml) were added for partitioning and the obtained organic layer was analyzed by the LC-IS method. As a result, the amount of 2-[4-(trifluoromethyl)phenyl]benzaldehyde in the organic layer was 0.65 g (yield:81.9%).

When the reaction was carried out in the same manner as above except the use of paraformaldehyde, the amount of the obtained 2-[4-(trifluoromethyl)phenyl]benzaldehyde was 0.499 g (yield:63.0%).

### Example 8 (production of compound [III] from compound [II]

In a 500 ml four-neck flask were added 2-[4-(trifluoromethyl)phenyl]toluene (40 g, 169.3 mmol) and monochlorobenzene (200 g) and the mixture was heated to 60°C. AIBN (0.28 g, 1.7 mmol) was added and bromine (31.2 g, 195.2 mmol) was added dropwise over 5 hr at 60°C - 65°C. After the dropwise addition, the mixture was stirred for one more hour and concentrated under reduced pressure. Crystallization from n-heptane gave 2-[4-(trifluoromethyl)phenyl]benzyl bromide (16.2 g, 51.4 mmol, liquid chromatography(LC) purity:99.8%, yield:30%) as white crystals.
¹H-NMR(400MHz,CDCl₃)δ:
7.72(d,J=7.8Hz,2H), 7.57(d,J=7.8Hz,2H), 7.55(dd,J=7.3,1.5Hz,lH),
7.39(ddt,J=7.3,7.3,1.5Hz,2H), 7.23(dd, J=7.3, 1.5Hz, 1H), 4.40(s,2H).

According to the method of the present invention, compound [V] represented by 2-[4-(trifluoromethyl)phenyl]benzoic acid useful as an intermediate for medicaments and agrochemicals, can be produced by an economical, simple and easy, efficient and industrially useful method. Further, the method of the present invention enables production of compound [IV], which is a synthetic intermediate for compound [V], by an economical, simple and easy, and industrially useful method.

Moreover, the method of the present invention enables economical production of compound [II], useful as an intermediate for medicaments and agrochemicals and useful as a starting material of compound [III]. According to the inventive method, an expensive catalyst is not used but economical compound [I] is used as a starting material along with an organometallic compound having a tolyl group, which can be prepared easily, as a compound to react with. As a result, the method enables production in a high yield by a simple and easy, and industrially utilizable method.

The present invention uses an organometallic compound instead of a silane derivative to avoid complicated synthesis of silane derivatives. Therefore, the method is industrially advantageous.

## Claims

1. A production method of α,α,α-trifluoromethylphenyl-substituted benzoic acid, which comprises the steps of
(c) reacting α,α,α-trifluoromethylphenyl-substituted benzyl halide of the formula [III] 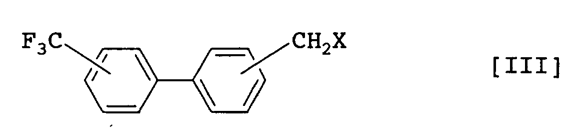 wherein X is a halogen atom, and hexamethylenetetramine under heating to give α,α,α-trifluoromethylphenyl-substituted benzaldehyde of the formula [IV] 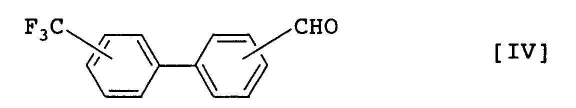 and
(d) oxidizing the α,α,α-trifluoromethylphenyl-substituted benzaldehyde with a halous acid salt or a ruthenium compound as an oxidizing agent to give α,α,α-trifluoromethylphenyl-substituted benzoic acid of the formula [V]

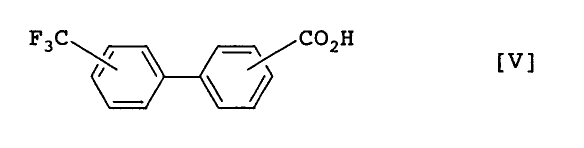

2. The production method of claim 1, further comprising adding formaldehyde or a polymer thereof in the step (c).

3. The production method of claim 1, further comprising adding a formaldehyde polymer in the step (c).

4. The production method of claim 2 or 3, wherein formaldehyde or a polymer thereof is added in an amount of 0.1 g- 0.57 g per 1 g of α,α,α-trifluoromethylphenyl-substituted benzyl halide of the formula [III].

5. The production method of claim 1, wherein the reaction of step (c) is carried out at pH 3.0 - 6.5.

6. The production method of claim 1, further comprising adding acetic acid in step (c) in an amount of 1 ml - 5 ml per 1 g of α,α,α-trifluoromethylphenyl-substituted benzyl halide of formula [III].

7. The production method of claim 1, wherein the reaction of step (d) is carried out using a halous acid salt as the oxidizing agent and in a mixed solvent of t-butanol and water.

8. The production method of claim 1, wherein the halous acid salt of step (d) is a chlorite.

9. The production method of claim 8, wherein the chlorite is sodium chlorite.

10. The production method of claim 1, wherein the reaction of step (d) is carried out using a halous acid salt as the oxidizing agent and in the presence of sulfamic acid.

11. The production method of claim 1, wherein the reaction of step (d) is carried out using a ruthenium compound as the oxidizing agent and in the presence of at least one member selected from the group consisting of sodium hypochlorite, sodium periodate and sodium bromate.

12. The production method of claim 1, wherein the reaction of step (d) is carried out using a ruthenium compound as the oxidizing agent, in the presence of a phase transfer catalyst and in a two-phase system of water and a solvent immiscible with water.

13. The production method of claim 1, wherein α,α,α-trifluoromethylphenyl-substituted benzyl halide of the formula [III] is 2-[4-(trifluoromethyl)phenyl]benzyl bromide.

14. The production method of claim 1, further comprising a step of (b) halogenating α,α,α-trifluoromethylphenyl-substituted toluene of the formula [II] 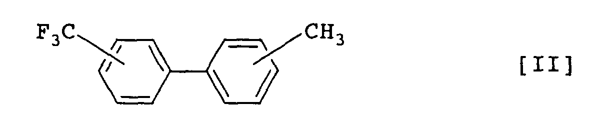 to give α,α,α-trifluoromethylphenyl-substituted benzyl halide of the formula [III], before the steps (c) and (d).

15. The production method of claim 14, further comprising a step of
(a) cross-coupling α,α,α-trifluoromethyl-substituted phenyl chloride of the formula [I] 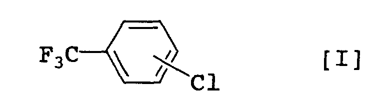 and an organometallic compound having a tolyl group, in the presence of a catalyst to give α,α,α-trifluoromethylphenyl-substituted toluene of the formula [II], before step (b).

16. The production method of claim 15, wherein the organometallic compound having a tolyl group is an organometallic compound having a 2-methylphenyl group.

17. A production method of 2-methylphenyl-substituted α,α,α-trifluorotoluene of the formula [ii] 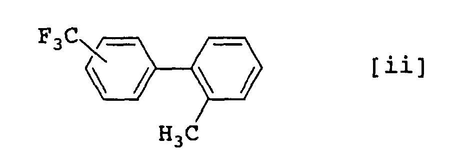 , which comprises cross-coupling α,α,α-trifluoromethyl-substituted phenyl chloride of the formula [I] 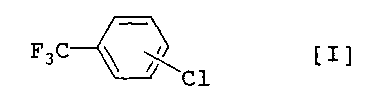 and an organometallic compound having a 2-methylphenyl group, in the presence of a catalyst.

18. The production method of claim 17, wherein the α,α,α-trifluoromethyl-substituted phenyl chloride of the formula [I] is 4-(trifluoromethyl)phenyl chloride.

19. The production method of claim 17, wherein the organometallic compound is 2-methylphenylmagnesium halide.

20. The production method of claim 17, wherein the catalyst is a nickel catalyst.

21. The production method of claim 17, wherein the catalyst is bis(triphenylphosphine)nickel(II) dichloride.

22. The production method of claim 17, wherein the catalyst is a nickel catalyst and a cocatalyst is used in addition to the catalyst.

23. The production method of claim 22, wherein the cocatalyst is [1] a zinc salt, or [2] a combination of a zinc salt and at least one member selected from the group consisting of a polar aprotic solvent and tertiary amine.

24. The production method of claim 22, wherein the cocatalyst is a combination of a zinc salt and a polar aprotic solvent.

25. The production method of claim 22, wherein the cocatalyst is a combination of a zinc salt and tertiary amine.

26. The production method of any of claims 23-25, wherein the zinc salt is at least one member selected from the group consisting of zinc chloride and zinc bromide.

27. The production method of claim 23 or 24, wherein the polar aprotic solvent is at least one member selected from the group consisting of N,N-dimethylformamide, dimethyl sulfoxide, N,N-dimethylacetamide and N-methyl-2-pyrrolidone.

28. The production method of claim 23 or 25, wherein the tertiary amine is at least one member selected from the group consisting of N,N,N',N'-tetramethylethylenediamine, N,N,N',N',N"-pentamethyldiethylenetriamine and dimethylaniline and pyridine.

29. The production method of claim 22, wherein the catalyst is bis(triphenylphosphine)nickel(II) dichloride and the cocatalyst is a combination of zinc chloride and N,N-dimethylacetamide.

30. The production method of claim 22, wherein the catalyst is bis(triphenylphosphine)nickel(II) dichloride and the cocatalyst is a combination of zinc chloride and N,N,N',N'-tetramethylethylenediamine.
